(19) **Europäisches Patentamt / European Patent Office / Office européen des brevets**

(11) **EP 2 818 109 B1**

(12) **EUROPEAN PATENT SPECIFICATION**

(45) Date of publication and mention
of the grant of the patent:
**31.08.2016 Bulletin 2016/35**

(51) Int Cl.:
*A61B 5/1455* (2006.01)     *A61H 31/00* (2006.01)
*G01N 21/35* (2014.01)

(21) Application number: **12869188.8**

(22) Date of filing: **25.12.2012**

(86) International application number:
**PCT/JP2012/083509**

(87) International publication number:
**WO 2013/125148 (29.08.2013 Gazette 2013/35)**

(54) **CONCENTRATION MEASUREMENT DEVICE AND CONCENTRATION MEASUREMENT METHOD**

KONZENTRATIONSMESSVORRICHTUNG UND KONZENTRATIONSMESSVERFAHREN

DISPOSITIF DE MESURE DE CONCENTRATION ET PROCÉDÉ DE MESURE DE CONCENTRATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.02.2012 JP 2012034035**

(43) Date of publication of application:
**31.12.2014 Bulletin 2015/01**

(73) Proprietor: **Hamamatsu Photonics K.K.**
**Hamamatsu-shi, Shizuoka 435-8558 (JP)**

(72) Inventors:
• **OZAKI, Takeo**
**Hamamatsu-shi**
**Shizuoka 435-8558 (JP)**
• **SUZUKI, Susumu**
**Hamamatsu-shi**
**Shizuoka 435-8558 (JP)**

(74) Representative: **Hoffmann Eitle**
**Patent- und Rechtsanwälte PartmbB**
**Arabellastraße 30**
**81925 München (DE)**

(56) References cited:
**WO-A1-2006/006143     JP-A- 2008 167 818
JP-A- 2009 125 402     US-A1- 2005 101 889**

• **N. NAGDYMAN: "Cerebral oxygenation measured by near-infrared spectroscopy during circulatory arrest and cardiopulmonary resuscitation", BRITISH JOURNAL OF ANAESTHESIA, vol. 91, no. 3, 1 September 2003 (2003-09-01), pages 438-442, XP055208245, ISSN: 0007-0912, DOI: 10.1093/bja/aeg181**
• **SUSUMU SUZUKI ET AL: "<title>Tissue oxygenation monitor using NIR spatially resolved spectroscopy</title>", PROCEEDINGS OF SPIE, vol. 3597, 15 July 1999 (1999-07-15), pages 582-592, XP055189471, ISSN: 0277-786X, DOI: 10.1117/12.356862**
• **ANTTI KMRINEN ET AL: "Quality controlled manual chest compressions and cerebral oxygenation during in-hospital cardiac arrest", RESUSCITATION, ELSEVIER, IE, vol. 83, no. 1, 8 September 2011 (2011-09-08), pages 138-142, XP028340672, ISSN: 0300-9572, DOI: 10.1016/J.RESUSCITATION.2011.09.011 [retrieved on 2011-09-28]**

## Description

### Technical Field

The present invention relates to a concentration measurement apparatus and a concentration measurement method

### Background Art

An example of a device for noninvasively measuring hemoglobin concentration information inside a living body is described in Patent Document 1. With this device, light is made incident inside the living body, and thereafter, light scattered inside the living body is detected by each of a plurality of photodiodes. Then, based on the intensities of the detected light components, a rate of change of the detected light amount in the direction of distance from the light incidence point is calculated. Hemoglobin oxygen saturation is calculated based on a predetermined relationship of the rate of change of the detected light amount and the light absorption coefficient. Also, based on a predetermined relationship of the temporal change of the rate of change of the detected light amount and the temporal change of the light absorption coefficient, respective concentration changes of oxygenated hemoglobin ($O_2Hb$), deoxygenated hemoglobin (HHb), and total hemoglobin (cHb) are calculated.

N. Nagdyman "Cerebral oxygenation measured by near-infrared spectroscopy during circulatory arrest and cardiopulmonary resuscitation", BRITISH JOURNAL OF ANAESTHESIA, vol. 91, no. 3, 1 September 2003, pages 438-442, discloses the use of the NIRO 300 (Hamamatsu Photonics, Japan) near-infrared spectroscopy monitoring apparatus for measuring a temporal relative change amount of oxygenated hemoglobin concentration, that varies due to repetition of chest compression, in a head.

### Citation List

### Patent Literature

Patent Document 1: Japanese Patent Application Laid-Open No.

### Non Patent Literature

Non-Patent Document 1: Susumu Suzuki, et al., "Tissue oxygenation monitor using NIR spatially resolved spectroscopy," Proceedings of SPIE 3597, pp. 582-592

### Summary of Invention

### Technical Problem

The primary patients in the emergency medical field in recent years are those suffering cardiopulmonary arrest outside a hospital. The number of out-of-hospital cardiopulmonary arrest persons exceeds 100 thousand per year, and emergency medical care of these persons is a major social demand. An essential procedure for out-of-hospital cardiopulmonary arrest persons is chest compression performed in combination with artificial respiration. Chest compression is an act where the lower half of the sternum is cyclically compressed by another person's hands to apply an artificial pulse to the arrested heart. A primary object of chest compression is to supply blood oxygen to the brain of the cardiopulmonary arrest person. Whether or not chest compression is being performed appropriately thus has a large influence on the life or death of the cardiopulmonary arrest person. Methods and devices that are useful for objectively judging whether or not chest compression is being performed appropriately are thus being demanded.

Also, although it is desirable for chest compression to be performed continuously, it may have to be interrupted due to unavoidable matters, such as a necessary procedure, change of chest compression performer, etc. A phenomenon that must be noted in such cases is the change of brain oxygenation state due to the interruption of chest compression. For example, the oxygenation state gradually decreases (degrades) due to the oxygen consumption that accompanies brain metabolism during the interruption. Or, if the brain oxygenation state hardly changes even when the chest compression is interrupted, significant lowering of the brain metabolism may be considered. It is thus desired that the chest compression performer, etc., be able to check the brain oxygenation state during the interruption of chest compression.

The present invention has been made in view of the above problem, and an object thereof is to provide a concentration measurement apparatus and a concentration measurement method that enable a chest compression performer, etc., to easily check the brain oxygenation state during interruption of chest compression.

**Solution to Problem**

In order to solve the above-described problem, a concentration measurement apparatus according to the present invention is a concentration measurement apparatus for measuring a temporal relative change amount of oxygenated hemoglobin concentration, that varies due to repetition of chest compression, in a head, and includes a light incidence section making measurement light incident on the head, a light detection section detecting the measurement light that has propagated through the interior of the head and generating a detection signal in accordance with the intensity of the detected measurement light, a calculation section determining, based on the detection signal, the temporal relative change amount of the oxygenated hemoglobin concentration and performing a filtering process of removing frequency components less than a predetermined frequency from frequency components contained in the relative change amount, and a display section displaying first time series data indicating the filtering-processed relative change amount of the oxygenated hemoglobin concentration, and where the calculation section judges, based on the detection signal, whether or not chest compression is being performed and, if chest compression is not performed for a predetermined time, the display section switches from displaying the first time series data to displaying second time series data indicating the temporal relative change amount of the oxygenated hemoglobin concentration that contains frequency components less than the predetermined frequency.

Further, a concentration measurement method according to the present invention is a concentration measurement method of measuring a temporal relative change amount of oxygenated hemoglobin concentration, that varies due to repetition of chest compression, in a head, and includes a light incidence step of making measurement light incident on the head, a light detection step of detecting the measurement light that has propagated through the interior of the head and generating a detection signal in accordance with the intensity of the detected measurement light, a calculation step of determining, based on the detection signal, the temporal relative change amount of the oxygenated hemoglobin concentration and performing a filtering process of removing frequency components less than a predetermined frequency from frequency components contained in the relative change amount, and a display step of displaying first time series data indicating the filtering-processed relative change amount of the oxygenated hemoglobin concentration, and where, in the calculation step, whether or not chest compression is being performed is judged based on the detection signal and, if chest compression is not performed for a predetermined time, switching from displaying the first time series data to displaying second time series data indicating the temporal relative change amount of the oxygenated hemoglobin concentration that contains frequency components less than the predetermined frequency is performed in the display step.

Measurement of a relative change amount of oxygenated hemoglobin concentration in the head at a frequency sufficiently higher than the heartbeat frequency using a concentration measurement apparatus that uses near-infrared light reveals that, in chest compression, certain changes occur in the oxygenated hemoglobin concentration of the interior of the head (that is, the brain) each time the sternum is compressed cyclically. This phenomenon is considered to be due to variation in blood flow within the brain by the chest compression and may be usable as a material for objectively judging whether or not chest compression is being performed appropriately. However, the amplitude of such a concentration change (for example, of approximately 1 μmol) due to chest compression is extremely small in comparison to the amplitudes of changes (normally of not less than several μmol) of even longer cycle that occur in a normally active state of a healthy person or in a state where various procedures are being performed on a cardiopulmonary arrest person. It is thus extremely difficult to observe the variations due to chest compression if simply values corresponding to the oxygenated hemoglobin concentration are measured.

Therefore, with the above-described concentration measurement apparatus and concentration measurement method, in addition to determining the temporal relative change amount of the oxygenated hemoglobin concentration, the frequency components less than the predetermined frequency are removed from the frequency components contained in the relative change amount in the calculation section or the calculation step. Normally, the cycle of concentration change due to chest compression (that is, the preferable compression cycle of the chest compression process) is shorter than the cycle of the primary concentration change in the state where various procedures are being performed on a cardiopulmonary arrest person. Therefore, by removing the low frequency components (that is, the long cycle components) from the measured relative change amount as in the above-described concentration measurement apparatus and concentration measurement method, information on concentration changes due to chest compression can be extracted favorably. Therefore, based on this information, a chest compression performer can objectively judge whether or not chest compression is being performed appropriately. It thus becomes possible for the performer to perform or maintain the chest compression more appropriately.

Also, as described above, it is desirable that the chest compression performer, etc., be able to check the brain oxygenation state when the chest compression is interrupted. Therefore, with the above-described concentration measurement apparatus and concentration measurement method, first, the calculation section judges, based on the detection signal, whether or not chest compression is being performed. Then, if chest compression is not performed for the predetermined time, the display section switches from displaying the first time series data that indicate the relative change amount from which the long cycle components have been removed (that is, indicate the concentration variation due to chest compression) to displaying second time series data that indicate the relative change amount containing the long cycle components (that is, mainly indicate the brain oxygenation state). Therefore, by the concentration measurement apparatus and concentration measurement method described above, the performer, etc., can easily check the brain oxygenation state during the interruption of chest compression.

The "filtering process of removing frequency components less than a predetermined frequency" in the above-described concentration measurement apparatus and concentration measurement method refers to a process of decreasing the proportion of frequency components less than the predetermined frequency until the frequency component due to chest compression appears at a sufficiently recognizable level, and is not restricted to completely removing the frequency components less than the predetermined frequency.

**Advantageous Effects of Invention**

In accordance with the concentration measurement apparatus and concentration measurement method according to the present invention, a chest compression performer, etc., can easily check the brain oxygen state during interruption of chest compression.

**Brief Description of Drawings**

[FIG. 1] FIG 1 is a conceptual diagram of a concentration measurement apparatus according to an embodiment.
[FIG 2] FIG. 2 includes (a) a plan view of a configuration of a probe, and (b) a sectional side view taken along line II-II of (a).
[FIG 3] FIG 3 is a block diagram of a configuration example of the concentration measurement apparatus.
[FIG 4] FIG 4 is a flowchart of a concentration measurement method according to an embodiment.
[FIG. 5] FIG 5 is a flowchart of the concentration measurement method according to the embodiment.
[FIG. 6] FIG 6 includes (a) a diagram of incidence timings of laser light beams of wavelengths $\lambda_1$ to $\lambda_3$, and (b) a diagram of output timings of digital signals from an A/D converter circuit.
[FIG. 7] FIG 7 is a graph of filter characteristics of a digital filter.
[FIG. 8] FIG 8 is a graph of results of using the digital filter having the characteristics shown in FIG 7 to remove frequency components less than a predetermined frequency from frequency components contained in a temporal relative change amount ($\Delta O_2 Hb$) of oxygenated hemoglobin to thereby extract a temporal variation component due to a spontaneous heartbeat that simulates the repetition of chest compression.
[FIG. 9] FIG. 9 is a graph of results of using a filtering process by smoothing to remove frequency components less than a predetermined frequency from frequency components contained in a temporal relative change amount ($\Delta cHb$) of total hemoglobin to thereby extract a temporal variation component due to a spontaneous heartbeat that simulates the repetition of chest compression.
[FIG. 10] FIG. 10 shows diagrams for describing concepts of a filtering process by which maximal portions or minimal portions of a variation are uniformized.
[FIG. 11] FIG 11 is a diagram of an example of time series data displayed on a display section.
[FIG. 12] FIG 12 is a diagram of an example of frequency characteristics of a band-pass filter.
[FIG. 13] FIG 13 is a graph of time series data of a temporal relative change amount of total hemoglobin concentration before the filtering process by the band-pass filter is performed, and time series data after the filtering process.
[FIG. 14] FIG 14 is a graph of compression time series data when chest compression is interrupted.
[FIG. 15] FIG 15 is a graph of interruption time series data displayed on the display section as a modified example.
[FIG. 16] FIG 16 is a diagram of frequency characteristics of a low-pass filter.
[FIG 17] FIG 17 shows graphs that schematically show time series data displayed on the display section.
[FIG 18] FIG. 18 is a diagram of an example of a graph displayed on the display section.

**Description of Embodiments**

Hereinafter, an embodiment of a concentration measurement apparatus and a concentration measurement method according to the present invention will be described in detail with reference to the accompanying drawings. In the description of the drawings, elements that are the same are provided with the same reference symbols, and redundant description is omitted.

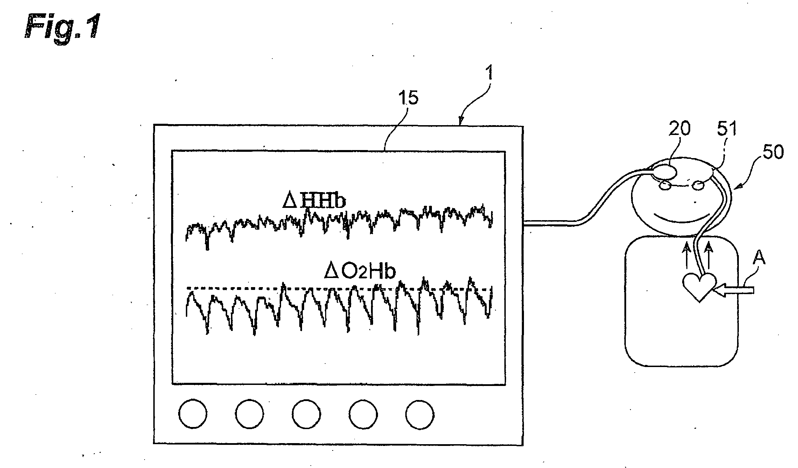

FIG. 1 is a conceptual diagram of a concentration measurement apparatus 1 according to an embodiment of the present invention. To provide material for objectively judging whether or not chest compression (arrow A in the figure) is being performed appropriately on a cardiopulmonary arrest person 50, the concentration measurement apparatus 1 measures respective temporal variations (relative change amounts) from initial amounts of total hemoglobin (cHb) concentration, oxygenated hemoglobin ($O_2Hb$) concentration, and deoxygenated hemoglobin (HHb) concentration of the head 51 that vary due to repeated chest compression, and displays the measurement results on a display section 15 to notify a person performing the chest compression.

The concentration measurement apparatus 1 makes light beams of predetermined wavelengths ($\lambda_1$, $\lambda_2$, and $\lambda_3$) be incident on a predetermined light incidence position from a probe 20 fixed to the head 51, and detects intensities of light components emitted from predetermined light detection positions on the head 51 to examine the effects of the oxygenated hemoglobin ($O_2Hb$) and the deoxygenated hemoglobin (HHb) on the light, and based thereon, repeatedly calculates the temporal relative change amounts of the oxygenated hemoglobin ($O_2Hb$) and the deoxygenated hemoglobin (HHb). Also, a filtering process is applied to time series data that are the calculation results to thereby remove low frequency components and extract a short-cycle temporal variation component due to the repetition of chest compression, and the temporal variation component is displayed in a visible manner. As the light of predetermined wavelengths, for example, near-infrared light is used.

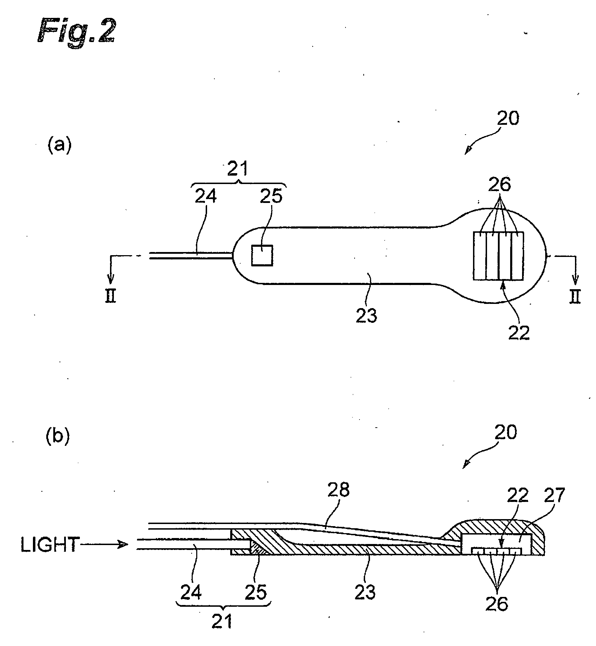

(a) in FIG. 2 is a plan view of a configuration of a probe 20. Further, (b) in FIG 2 is a sectional side view taken along line II-II of (a) in FIG 2. The probe 20 has a light incidence section 21 and a light detection section 22. The light incidence section 21 and the light detection section 22 are disposed with an interval, for example, of 5 cm from each other, and are practically integrated by a holder 23 made of flexible, black silicone rubber. Here, the interval suffices to be not less than approximately 3 to 4 cm.

The light incidence section 21 includes an optical fiber 24 and a prism 25, and has a structure that makes the measurement light, transmitted from a main unit section 10 of the concentration measurement apparatus 1, incident substantially perpendicularly on the skin of the head. The measurement light is, for example, a laser light beam of pulse form, and is transmitted from the main unit section 10.

The light detection section 22 detects measurement light components that have propagated through the interior of the head, and generates detection signals that are in accordance with the intensities of the measurement light components. The light detection section 22 is, for example, a one-dimensional photosensor having an array of N photodetection elements 26 aligned in a direction of distance from the light incidence section 21. Also, the light detection section 22 further has a pre-amplifier section 27 that integrates and amplifies photocurrents output from the photodetection elements 26. By this configuration, weak signals can be detected with high sensitivity to generate detection signals, and the signals can be transmitted via a cable 28 to the main unit section 10. Here, the light detection section 22 may instead be a two-dimensional photosensor, or may be configured by a charge-coupled device (CCD). The probe 20 is, for example, fixed by an adhesive tape or a stretchable band, etc., onto a forehead portion without hair.

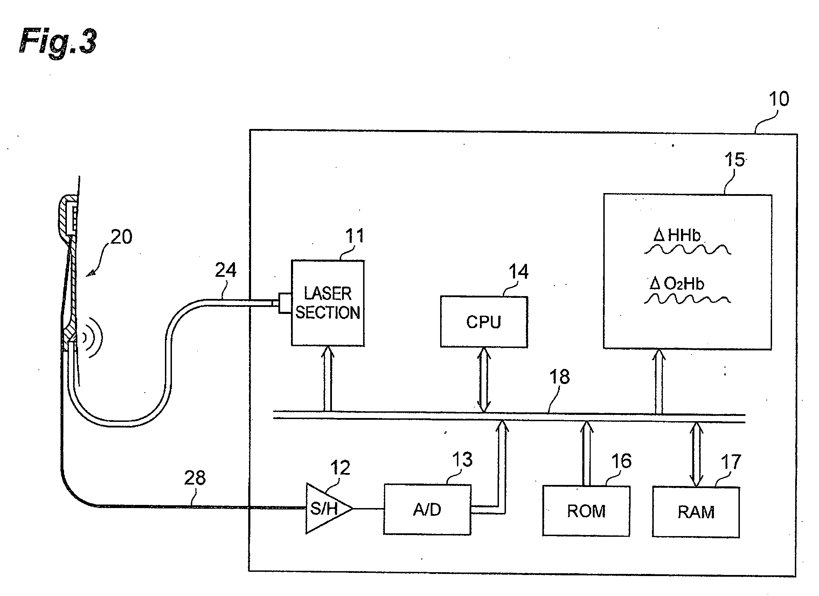

FIG 3 is a block diagram of a configuration example of the concentration measurement apparatus 1. The concentration measurement apparatus 1 shown in FIG. 3 includes the main unit section 10 in addition to the probe 20 described above. The main unit section 10 includes a light emitting section 11, a sample hold circuit 12, an A/D converter circuit 13, a CPU 14, a display section 15, a ROM 16, a RAM 17, and a data bus 18.

The light emitting section 11 is configured by a laser diode and a circuit that drives the laser diode. The light emitting section 11 is electrically connected to the data bus 18 and receives an instruction signal for instructing the driving of the laser diode from the CPU 14 that is similarly electrically connected to the data bus 18. The instruction signal contains information on the light intensity, wavelength (for example, a wavelength among wavelengths $\lambda_1$, $\lambda_2$, and $\lambda_3$), etc., of the laser light output from the laser diode. The light emitting section 11 drives the laser diode based on the instruction signal received from the CPU 14 and outputs laser light to the probe 20 via the optical fiber 24. Here, the light emitting element of the light emitting section 11 does not have to be a laser diode, and suffices to be an element that can successively output light beams of a plurality of wavelengths in the near-infrared region. Also, an LED or other light emitting diode that is built into the probe 20 may be used as the light incidence section 21.

The sample hold circuit 12 and the A/D converter circuit 13 input the detection signals transmitted via the cable 28 from the probe 20 and perform holding and conversion of the signals to digital signals that are then output to the CPU 14. The sample hold circuit 12 simultaneously holds the values of N detection signals. The sample hold circuit 12 is electrically connected to the data bus 18, and receives a sample signal, indicating the timing of holding of the detection signals, from the CPU 14 via the data bus 18. Upon receiving the sample signal, the sample hold circuit 12 simultaneously holds N detection signals input from the probe 20. The sample hold circuit 12 is electrically connected to the A/D converter circuit 13, and outputs each of the held N detection signals to the A/D converter circuit 13.

The A/D converter circuit 13 is means for converting the detection signals from analog signals to digital signals. The A/D convertor circuit 13 successively converts the N detection signals received from the sample hold circuit 12 into digital signals. The A/D convertor circuit 13 is electrically connected to the data bus 18 and outputs the converted detection signals to the CPU 14 via the data bus 18.

The CPU 14 is a calculation section in the present embodiment and, based on the detection signals received from the A/D converter circuit 13, calculates the temporal relative change amount of the oxygenated hemoglobin concentration ($\Delta O_2Hb$) contained in the interior of the head, and further calculates the required amounts among the temporal relative change amount of the deoxygenated hemoglobin concentration ($\Delta HHb$), and the temporal relative change amount of the total hemoglobin concentration ($\Delta cHb$), which is the sum of these amounts. Further, the CPU 14 applies a filtering process to the temporal relative change amounts ($\Delta O_2Hb$, $\Delta HHB$, $\Delta cHb$) to remove frequency components less than a predetermined frequency $f_0$ from frequency components contained in the amounts to thereby extract temporal variation components due to repetition of chest compression.

In the present embodiment, the "filtering process of removing frequency components less than a predetermined frequency $f_0$" refers to a process of decreasing the proportion of frequency components less than the predetermined frequency $f_0$ until the frequency component due to chest compression appears at a sufficiently recognizable level, and is not restricted to completely removing the frequency components less than the predetermined frequency $f_0$.

The CPU 14 transmits time series data indicating the change with time of the filtering-processed oxygenated hemoglobin concentration temporal relative change amount ($\Delta O_2Hb$) (the first time series data in the present embodiment, hereinafter referred to as compression time series data of the oxygenated hemoglobin concentration) to the display section 15 via the data bus 18. Further, the CPU 14 may also transmit time series data indicating the change with time of at least one of the filtering-processed deoxygenated hemoglobin concentration and total hemoglobin concentration temporal relative change amounts ($\Delta HHb$, $\Delta cHb$) (the third time series data in the present embodiment, hereinafter referred to as compression time series data of the deoxygenated hemoglobin concentration and total hemoglobin concentration) to the display section 15 via the data bus 18.

A method of calculating the temporal relative change amounts ($\Delta O_2Hb$, $\Delta HHb$, $\Delta cHb$) based on the detection signals and a method of the filtering process shall be described later.

Also, the CPU 14 judges, based on the detection signal obtained via the data bus 18, whether or not chest compression is being performed. For example, the CPU 14 judges that chest compression is not being performed (or is interrupted) when the amplitude of any of the filtering-processed hemoglobin concentration temporal relative change amounts ($\Delta O_2Hb$, $\Delta HHb$, $\Delta cHb$) drops below a predetermined proportion and this state is sustained for a predetermined time.

When the CPU 14 judges that chest compression is not being performed (is interrupted), the providing of the compression time series data related to the oxygenated hemoglobin concentration to the display section 15 is interrupted, and instead, time series data indicating the temporal relative change amount ($\Delta O_2Hb$) that contains frequency components less than the predetermined frequency $f_0$ (the second time series data in the present embodiment, hereinafter referred to as interruption time series data of the oxygenated hemoglobin concentration) are provided to the display section 15. Similarly, when the CPU 14 judges that chest compression is not being performed (is interrupted), the providing of the compression time series data of the deoxygenated hemoglobin concentration and total hemoglobin concentration to the display section 15 is interrupted, and time series data indicating the temporal relative change amounts ($\Delta HHb$, $\Delta cHb$) that contain frequency components less than the predetermined frequency $f_0$ (the fourth time series data in the present embodiment, hereinafter referred to as interruption time series data of the deoxygenated hemoglobin concentration and total hemoglobin concentration) are provided to the display section 15.

Here, the interruption time series data are, for example, data indicating the non-filtering-processed temporal relative change amount ($\Delta O_2Hb$, $\Delta HHb$, $\Delta cHb$). Or, the interruption time series data are, for example, data indicating values obtained by subtracting the filtering-processed temporal relative change amount ($\Delta O_2Hb$, $\Delta HHb$, $\Delta cHb$) from the non-filtering-processed temporal relative change amount ($\Delta O_2Hb$, $\Delta HHb$, $\Delta cHb$).

The display section 15 is electrically connected to the data bus 18 and displays the time series data transmitted from the CPU 14 via the data bus 18. That is, when chest compression is being performed, the display section 15 displays the compression time series data indicating the filtering-processed temporal relative change amount ($\Delta O_2Hb$, $\Delta HHb$, $\Delta cHb$) that are provided from the CPU 14. When the CPU 14 judges that chest compression is not being performed (is interrupted), the display section 15 switches from displaying the compression time series data to displaying the interruption time series data indicating the temporal relative change amount ($\Delta O_2Hb$, $\Delta HHb$, $\Delta cHb$) that contains frequency components less than the predetermined frequency $f_0$.

Also, in switching from displaying the compression time series data to displaying the interruption time series data, the display section 15 may retrospectively display interruption time series data obtained before the CPU 14 recognized that chest compression is not performed for the predetermined time. In this case, preferably, the CPU 14 calculates the oxygenated hemoglobin concentration interruption time series data or data corresponding to the interruption time series data even while chest compression is being performed, and successively stores the data in the RAM 17 (corresponding to a storage section in the present embodiment).

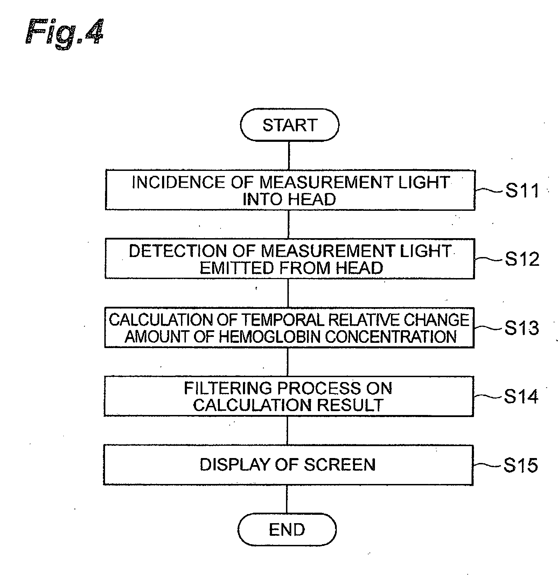

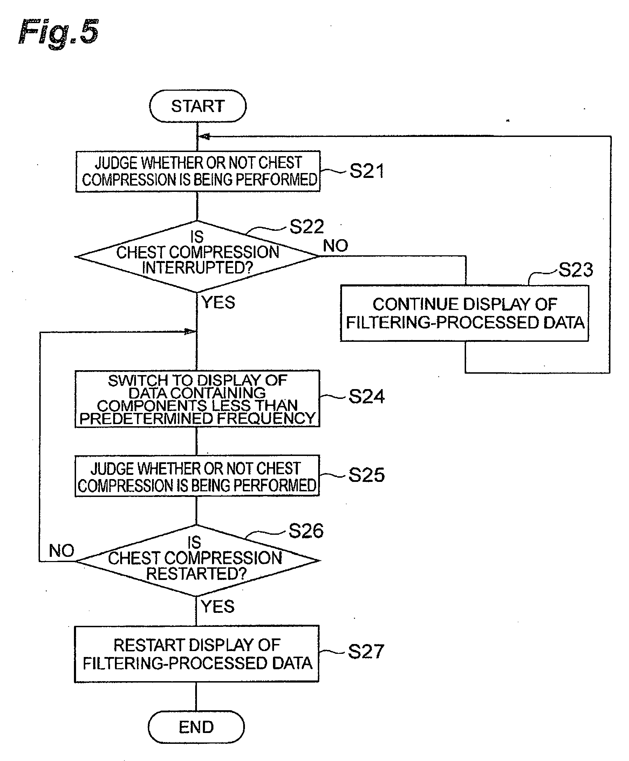

The operation of the concentration measurement apparatus 1 shall now be described. In addition, the concentration measurement method according to the present embodiment shall be described. FIG 4 and FIG 5 are flowcharts of the concentration measurement method according to the present embodiment.

First, the light emitting section 11 successively outputs the laser light beams of wavelengths $\lambda_1$ to $\lambda_3$ based on

the instruction signal from the CPU 14. The laser light beams propagate through the optical fiber 24, reach the light incidence position at the forehead portion, and enter inside the head from the light incidence position (light incidence step, S 11 of FIG. 4). The laser light beam made to enter inside the head propagates while being scattered inside the head and being absorbed by measurement object components, and parts of the light reach the light detection positions of the forehead portion. The laser light components that reach the light detection positions are detected by the N photodetection elements 26 (light detection step, S12 of FIG 4). Each photodetection element 26 generates a photocurrent in accordance with the intensity of the detected laser light component. These photocurrents are converted into voltage signals (detection signals) by the pre-amplifier section 27, and the voltage signals are transmitted to and held by the sample hold circuit 12 of the main unit section 10, and thereafter, converted to digital signals by the A/D converter circuit 13.

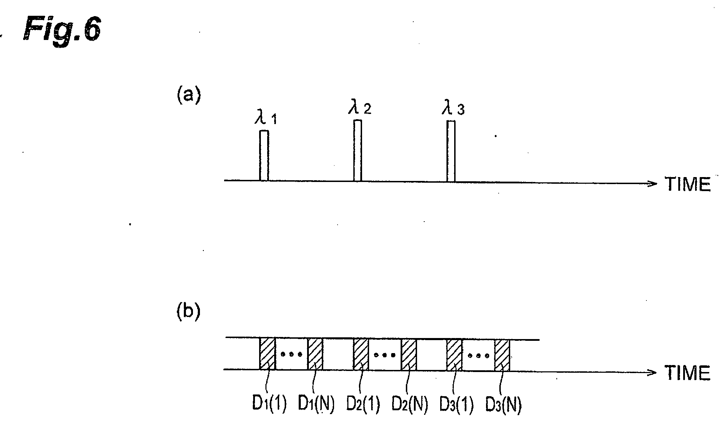

Here, (a) in FIG 6 is a diagram of incidence timings of the laser light beams of wavelengths $\lambda_1$ to $\lambda_3$, and (b) in FIG. 6 is a diagram of output timings of the digital signals from the A/D converter circuit 13. As shown in FIG 6, when the laser light of wavelength $\lambda_1$ is made incident, N digital signals $D_1(1)$ to $D_1(N)$ corresponding to the N photodetection elements 26 are obtained successively. Next, when the laser light of wavelength $\lambda_2$ is made incident, N digital signals $D_2(1)$ to $D_2(N)$ corresponding to the N photodetection elements 26 are obtained successively. Thus, $(3 \times N)$ digital signals $D_1(1)$ to $D_3(N)$ are output from the A/D converter circuit 13.

Subsequently, the CPU 14 calculates the hemoglobin oxygen saturation (TOI) based on the digital signals D(1) to D(N). Also, the CPU 14 uses at least one digital signal from the digital signals D(1) to D(N) to calculate the oxygenated hemoglobin concentration temporal relative change amount ($\Delta O_2Hb$), and also calculate, as necessary, either or both of the deoxygenated hemoglobin concentration temporal relative change amount ($\Delta HHb$) and the total hemoglobin concentration temporal relative change amount ($\Delta cHb$), which is the sum of these (calculation step, step S 13). Then, of the frequency components contained in the relative change amounts ($\Delta cHb$, $\Delta O_2Hb$, $\Delta HHb$), the frequency components less than the predetermined frequency $f_0$ are removed by a filtering process (calculation step, S 14 of FIG 4). Time series data (compression time series data) indicating the filtering-processed relative change amounts ($\Delta cHb$ $\Delta O_2Hb$, $\Delta HHb$) are displayed on the display section 15 (step S15 of FIG 4).

The above-described calculation performed by the CPU 14 in the calculation steps S 13 and S 14 shall now be described in detail.

If $D_{\lambda 1}(T_0)$ to $D\lambda_3(T_0)$ are values of the detection signals, respectively corresponding to the laser light wavelengths $\lambda_1$ to $\lambda_3$, at a time $T_0$ at a certain light detection position, and $D_{\lambda 1}(T_1)$ to $D_{\lambda 3}(T_1)$ are likewise values at a time $T_1$, the change amounts of the detected light intensities in the time $T_0$ to $T_1$ are expressed by the following formulas (1) to (3).

[Formula 1]

$$\Delta OD_1(T_1) = \log\left(\frac{D_{\lambda 1}(T_1)}{D_{\lambda 1}(T_0)}\right) \quad \cdots (1)$$

[Formula 2]

$$\Delta OD_2(T_1) = \log\left(\frac{D_{\lambda 2}(T_1)}{D_{\lambda 2}(T_0)}\right) \quad \cdots (2)$$

[Formula 3]

$$\Delta OD_3(T_1) = \log\left(\frac{D_{\lambda 3}(T_1)}{D_{\lambda 3}(T_0)}\right) \quad \cdots (3)$$

Here, in the formulas (1) to (3), $\Delta OD_1(T_1)$ is the temporal change amount of the detected light intensity of wavelength $\lambda_1$, $\Delta OD_2(T_1)$ is the change amount of the detected light intensity of wavelength $\lambda_2$, and $\Delta OD_3(T_1)$ is the temporal change amount of the detected light intensity of wavelength $\lambda_3$.

Further, if $\Delta O_2Hb(T_1)$ and $\Delta HHb(T_1)$ are the temporal relative change amounts of the concentrations of oxygenated hemoglobin and deoxygenated hemoglobin, respectively, in the period from time To to time $T_1$, these can be determined by the following formula (4).

[Formula 4]

$$\begin{pmatrix} \Delta O_2 Hb(T_1) \\ \Delta HHb(T_1) \end{pmatrix} = \begin{pmatrix} a_{11} & a_{12} & a_{13} \\ a_{21} & a_{22} & a_{23} \end{pmatrix} \begin{pmatrix} \Delta OD_1(T_1) \\ \Delta OD_2(T_1) \\ \Delta OD_3(T_1) \end{pmatrix} \quad \cdots (4)$$

Here, in the formula (4), the coefficients a11 to a23 are constants determined from absorbance coefficients of $O_2$Hb and HHb for light components of wavelengths $\lambda_1$, $\lambda_2$ and $\lambda_3$. Also, the temporal relative change amount $\Delta$cHb($T_1$) of the total hemoglobin concentration in the head can be determined by the following formula (5).
[Formula 5]

$$\Delta cHb(T_1) = \Delta O_2 Hb(T_1) + \Delta HHb(T_1) \quad \cdots (5)$$

The CPU 14 performs the above calculation on detection signals from one position among the N light detection positions to calculate the respective temporal relative change amounts ($\Delta O_2$Hb, $\Delta$HHb, $\Delta$cHb) of the oxygenated hemoglobin concentration, deoxygenated hemoglobin concentration, and total hemoglobin concentration. Further, the CPU 14 performs, for example, any of the following filtering processes on the temporal relative change amounts ($\Delta O_2$Hb, $\Delta$HHb, $\Delta$cHb) that have thus been calculated.

(1) Filtering Process by a Digital Filter

Let X(n) be a data string related to a temporal relative change amount ($\Delta O_2$Hb, $\Delta$HHb, or $\Delta$cHb) obtained at a predetermined cycle. Here, n is an integer. By multiplying the respective data of the data string X(n) by, for example, the following filter coefficients A(n), with n=0 being the time center, a non-recursive linear phase digital filter is realized.

$$A(0)=3/4$$

$$A(3)=A(-3)=-1/6$$

$$A(6)=A(-6)=-1/8$$

$$A(9)=A(-9)=-1/12$$

To describe in further detail, a delay operator for the data string X(n) is represented by the following formula (6). Here, f is the time frequency (units: 1/sec). Also, $\omega$ is the angular frequency and $\omega=2\pi f$. T is the cycle at which the data string X(n) is obtained and is set, for example, to a cycle of 1/20 seconds for measuring a variation waveform at approximately 150 times per minute (2.5 Hz).
[Formula 6]

$$e^{j\omega nT} = COS(\omega nT) + jSIN(\omega nT)$$
$$e^{-j\omega nT} = COS(\omega nT) - jSIN(\omega nT) \quad \cdots (6)$$

In this case, the digital filter characteristics when the above-described filter coefficients A(n) are used are described by the following formula (7).
[Formula 7]

$$R(\omega) = \frac{3}{4} - \frac{1}{6}(e^{-3j\omega T} + e^{+3j\omega T}) - \frac{1}{8}(e^{-6j\omega T} + e^{+6j\omega T}) - \frac{1}{12}(e^{-9j\omega T} + e^{+9j\omega T})$$

$$= \frac{3}{4} - \frac{1}{3}COS(3\omega T) - \frac{1}{4}COS(6\omega T) - \frac{1}{6}COS(9\omega T) \qquad \cdots (7)$$

The digital filter is thus expressed by a product-sum operation of the data string X(n) and the corresponding coefficients. Further, by converting the time frequency f in formula (7) to a time frequency F per minute (units: 1/min), the following formula (8) is obtained.

[Formula 8]

$$R(F) = \frac{3}{4} - \frac{1}{3}COS(\frac{3\pi}{600}F) - \frac{1}{4}COS(\frac{6\pi}{600}F) - \frac{1}{6}COS(\frac{9\pi}{600}F) \qquad \cdots (8)$$

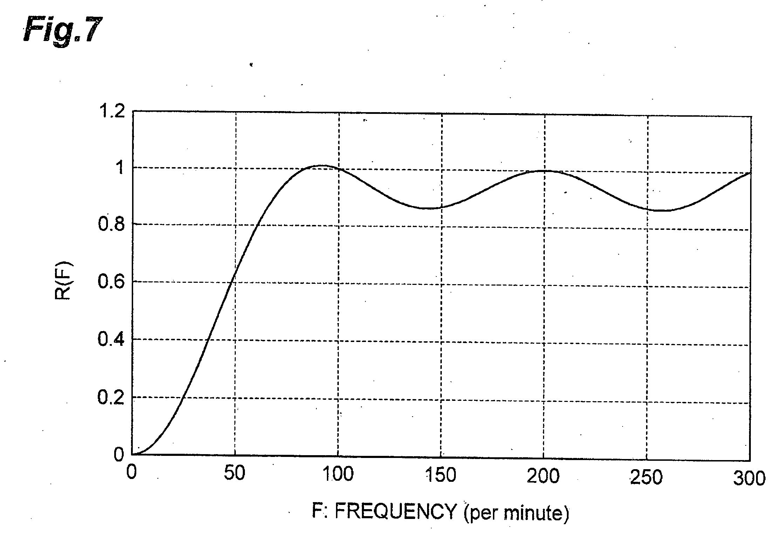

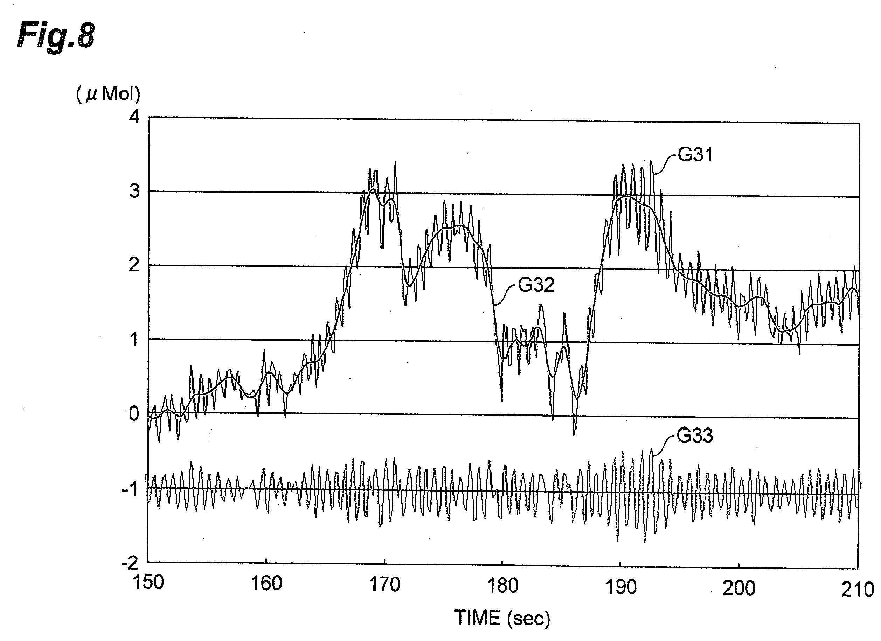

FIG. 7 is a graph of R(F) and shows the filter characteristics of the digital filter. In FIG 7, the horizontal axis represents the number of heartbeats per minute, and the vertical axis represents the value of R(F). Further, FIG 8 is a graph of results of using the digital filter shown in FIG 7 to remove (reduce) frequency components less than the predetermined frequency from the frequency components contained in the temporal relative change amount ($\Delta O_2 Hb$) of oxygenated hemoglobin to extract a temporal variation component due to a spontaneous heartbeat that simulates the repetition of chest compression. In FIG. 8, a graph G31 represents the relative change amount ($\Delta O_2 Hb$) before the filtering process, a graph G32 represents the long cycle components (frequency components less than the predetermined frequency) contained in the relative change amount ($\Delta O_2 Hb$) before the filtering process, and a graph G33 represents the relative change amount ($\Delta O_2 Hb$) after the filtering process. As shown in FIG. 8, by the above digital filter, the temporal variation component due to the spontaneous heartbeat or the repetition of chest compression can be extracted favorably.

(2) Filtering Process by a Smoothing Calculation (Least-Square Error Curve Fitting)

A least square error curve fitting using a high-order function (for example, a fourth-order function) is performed on a data string X(n), within the above-described data string X(n), that is obtained in a predetermined time (for example, 3 seconds, corresponding to 5 beats) before and after n=0 as the time center. The constant term of the high-order function obtained is then deemed to be a smoothed component (frequency component less than the predetermined frequency) at n=0. That is, by subtracting the smoothed frequency component from the original data X(0), the frequency component less than the predetermined frequency can be removed from the frequency components contained in the relative change amount to separate/extract the temporal variation component due to repeated chest compression.

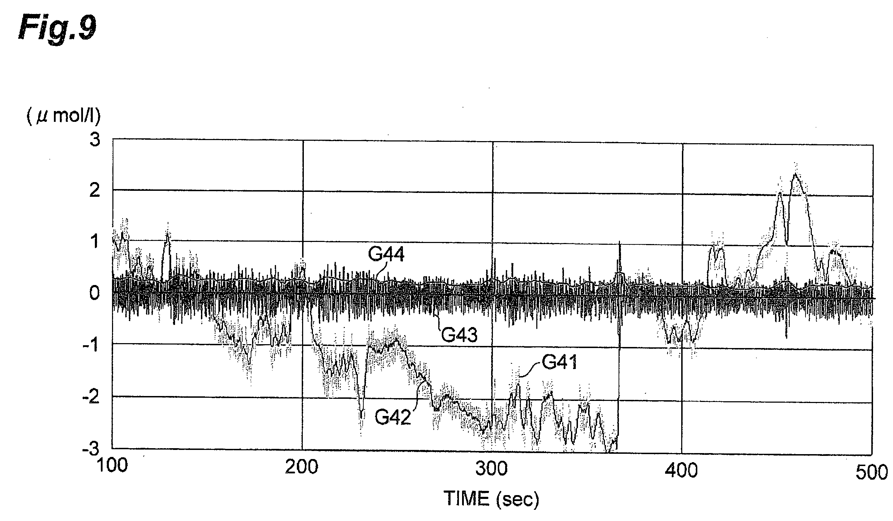

FIG. 9 is a graph of results of using such a filtering process to remove (reduce) frequency components less than the predetermined frequency from the frequency components contained in the temporal relative change amount ($\Delta c Hb$) of the total hemoglobin to extract a temporal variation component due to a spontaneous heartbeat that simulates the repetition of chest compression. In FIG 9, a graph G41 represents the relative change amount ($\Delta c Hb$) before the filtering process, a graph G42 represents the long cycle components (frequency components less than the predetermined frequency) contained in the relative change amount ($\Delta c Hb$) before the filtering process, a graph G43 represents the relative change amount ($\Delta c Hb$) after the filtering process, and a graph G44 indicates the 5-second average amplitudes in the relative change amount ($\Delta c Hb$) after the filtering process. As shown in FIG 9, by the filtering process by the above-described smoothing calculation, the temporal variation component due to the spontaneous heartbeat or the repetition of chest compression can be extracted favorably.

(3) Filtering Process of Uniformizing the Maximal Portions or Minimal Portions of Variation

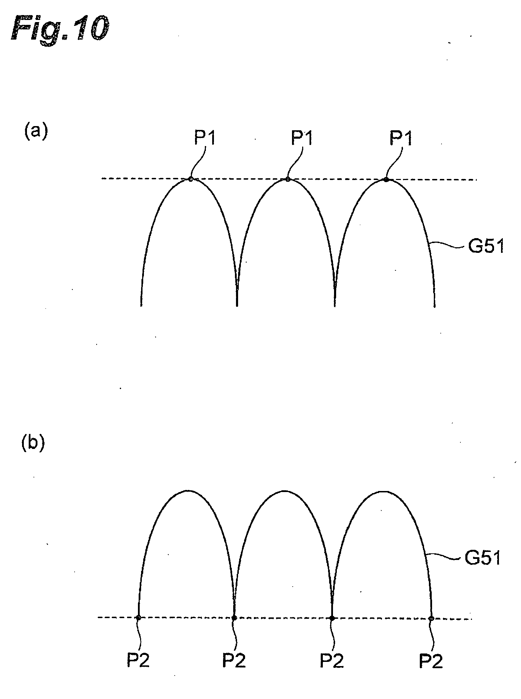

(a) in FIG. 10 and (b) in FIG. 10 are diagrams for describing the concepts of the present filtering process. In this filtering process, for example, the maximal values in the temporal variation of the relative change amount ($\Delta O_2 Hb$, $\Delta H Hb$, or $\Delta c Hb$) are determined, and by deeming the maximal values P1 in the temporal variation graph G51 to be of fixed value as shown in (a) in FIG 10, the frequency components less than the predetermined frequency that are contained in the relative change amount ($\Delta O_2 Hb$, $\Delta H Hb$, or $\Delta c Hb$) are removed. Or, for example, the minimal values in the temporal variation of the relative change amount ($\Delta O_2 Hb$, $\Delta H Hb$, or $\Delta c Hb$) are determined, and by deeming the minimal values P2 in the temporal variation graph G51 to be of fixed value as shown in (b) in FIG. 10, the frequency components less than the predetermined frequency that are contained in the relative change amount ($\Delta O_2Hb$, $\Delta HHb$, or $\Delta cHb$) are removed. By thus making either or both the maximal values P1 and minimal values P2 closer to fixed values, the temporal variation component due to the repetition of chest compression can be extracted favorably.

The concentration measurement method shown in FIG. 5 shall now be described. In the present embodiment, the CPU 14 judges whether or not chest compression is being performed in the calculation step S14 shown in FIG. 4, based on at least one of the respective hemoglobin concentration temporal relative change amounts ($\Delta O_2Hb$, $\Delta HHb$, $\Delta cHb$) calculated using the digital signals D(1) to D(N) shown in (b) in FIG 6 (step S21 of FIG. 5). For example, the CPU 14 judges that chest compression is not being performed (or is interrupted) when the amplitude of any of the filtering-processed hemoglobin concentration temporal relative change amounts ($\Delta O_2Hb$, $\Delta HHb$, $\Delta cHb$) drops below a predetermined proportion and this state is sustained for a predetermined time. If it is not judged that chest compression is interrupted (step S22 of FIG. 5: NO), the display of the compression time series data indicating the filtering-processed temporal relative change amounts ($\Delta O_2Hb$, $\Delta HHb$, $\Delta cHb$) provided from the CPU 14 is continued at the display section 15 (step S23 of FIG. 5).

Further, if the CPU 14 judges that chest compression is interrupted (step S22 of FIG. 5: YES), the display of the compression time series data is switched to the display of the interruption time series data indicating the temporal relative change amounts ($\Delta O_2Hb$, $\Delta HHb$, $\Delta cHb$) containing frequency components less than the predetermined frequency $f_0$ at the display section 15 (step S24 of FIG 5).

Thereafter, the CPU 14 judges the performing or non-performing of chest compression again based on at least one of the respective hemoglobin concentration temporal relative change amounts ($\Delta o_2Hb$, $\Delta HHb$, $\Delta cHb$) calculated using the digital signals D(1) to D(N) shown in (b) in FIG. 6 (step S25 of FIG. 5). If the CPU 14 judges that chest compression is restarted (step S26 of FIG. 5: YES), the display of the compression time series data indicating the filtering-processed temporal relative change amounts ($\Delta O_2Hb$, $\Delta HHb$, $\Delta cHb$) is restarted at the display section 15 (step S27 of FIG 5). As long as the CPU 14 does not judge that chest compression is restarted (step S26 of FIG. 5: NO), the display of the interruption time series data indicating the temporal relative change amounts ($\Delta O_2Hb$, $\Delta HHb$, $\Delta cHb$) containing frequency components less than the predetermined frequency $f_0$ is continued at the display section 15.

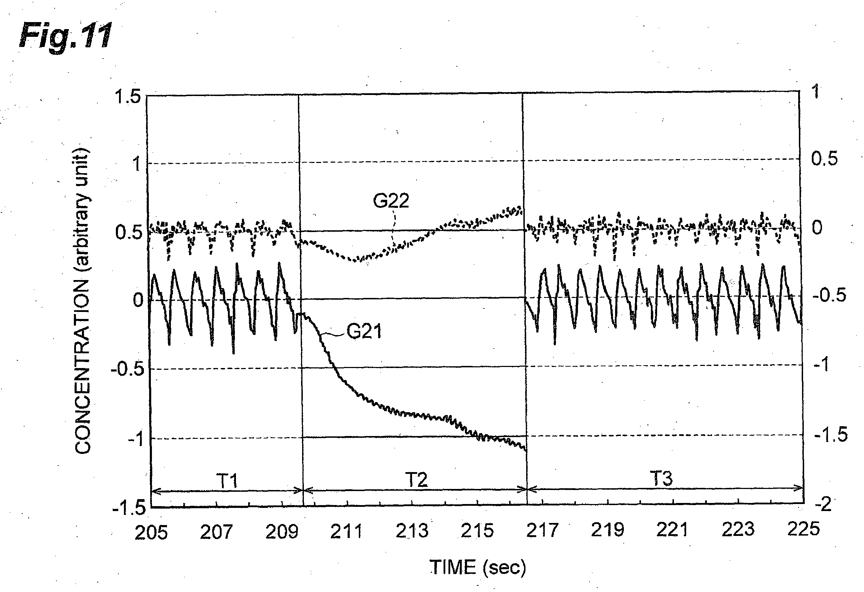

FIG. 11 is a diagram of an example of time series data displayed on the display section 15. In FIG. 11, periods T1 and T3 are periods in which the CPU 14 judged that chest compression is being performed, and a period T2 is a period in which the CPU 14 judged that chest compression is not performed (is interrupted). A graph G21 indicates time series data of the oxygenated hemoglobin concentration temporal relative change amount ($\Delta O_2Hb$), and a graph G22 indicates time series data of the deoxygenated hemoglobin concentration temporal relative change amount ($\Delta HHb$).

As shown in FIG 11, in the period T1, in which the CPU 14 does not judge that chest compression is interrupted, the respective time series data of the filtering-processed hemoglobin concentration temporal relative change amounts ($\Delta O_2Hb$, $\Delta HHb$) are displayed on the display section 15. These time series data mainly contain a cyclic change due to the chest compression.

In the period T2 after the CPU 14 judges that chest compression is interrupted, the time series data of the temporal relative change amounts ($\Delta O_2Hb$, $\Delta HHb$) that contain frequency components less than the predetermined frequency $f_0$ are displayed. These time series data mainly contain a change due to a change of the brain oxygenation state of the cardiopulmonary arrest person (normally, the amplitude of this change is greater than the amplitude of the change due to chest compression, and the cycle of this change is longer than the cycle of the change due to chest compression).

In the period T3 after the CPU 14 judged that chest compression has been restarted, the respective time series data of the filtering-processed hemoglobin concentration temporal relative change amounts ($\Delta O_2Hb$, $\Delta HHb$) are displayed again on the display section 15. These time series data mainly contain the cyclic change due to the chest compression.

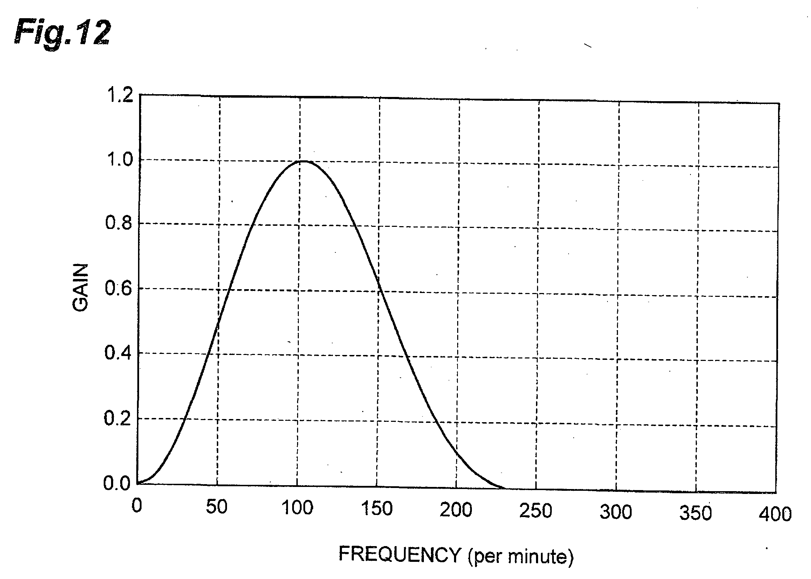

An example of a method by which the interruption and restarting of chest compression are judged by the CPU 14 shall now be described. The hemoglobin concentration temporal relative change amounts calculated using the digital signals D(1) to D(N) normally contain some amount of noise. To reduce the probability of erroneously judging the performing or non-performing of chest compression due to the noise, a filtering process is performed on the hemoglobin concentration temporal relative change amounts. For example, a band-pass filter having the frequency characteristics shown in FIG. 12 is used to take out just the fundamental wave components (with frequencies, for example, of 100 times/minute and thereabout) of the variations of the hemoglobin concentration temporal relative change amounts ($\Delta O_2Hb$, $\Delta HHb$, $\Delta cHb$) due to chest compression.

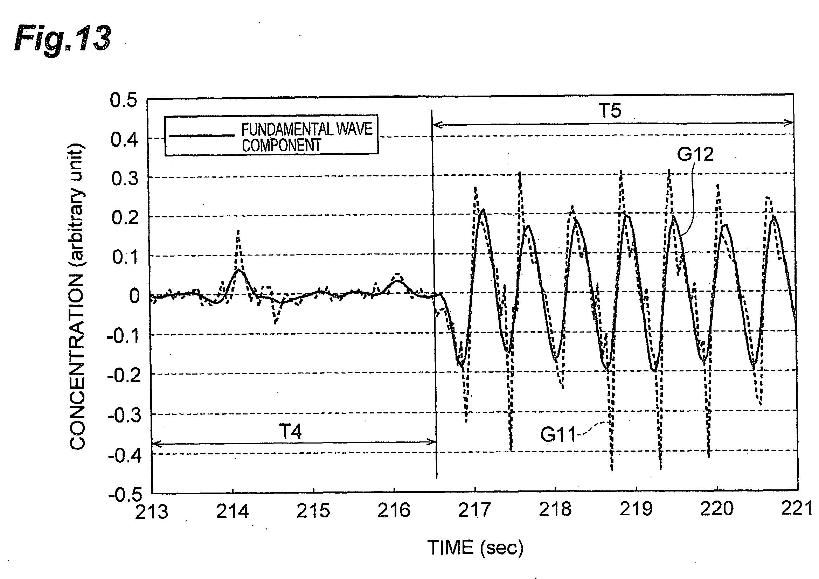

FIG 13 is a graph of time series data (graph G11) of the total hemoglobin concentration temporal relative change amount ($\Delta cHb$) before performing such a filtering process, and time series data (graph G12) of $\Delta cHb$ after the filtering process. In FIG. 13, chest compression is not performed in a period T4 of the first half, and chest compression is restarted in a period T5 of the latter half. As indicated by the graph G11, the temporal relative change amount ($\Delta cHb$) before performing the filtering process contains much noise and whether or not chest compression is being performed may be judged erroneously. On the other hand, as indicated by the graph G12, with the temporal relative change amount ($\Delta$cHb) after performing the filtering process, the noise is reduced effectively and the variation component due to the chest compression (fundamental wave component) appears more clearly.

The CPU 14 judges that chest compression is being performed when the amplitude of the fundamental wave component obtained, for example, by such a filtering process is no less than a predetermined value (to give one example, 0.1). Further, the CPU 14 judges that chest compression is interrupted when, after the state in which chest compression is being performed, a state, in which the amplitude of the fundamental wave component is less than the predetermined value, is sustained for no less than a predetermined time. Further, the CPU 14 judges that chest compression is restarted when, after the state in which the chest compression is interrupted, it is determined that chest compression is performed once (or several times).

The effects of the concentration measurement apparatus 1 and the concentration measurement method according to the present embodiment with the above configurations shall now be described. With the concentration measurement apparatus 1 and the concentration measurement method, in addition to the CPU 14 determining the oxygenated hemoglobin concentration temporal relative change amount ($\Delta O_2$Hb), the frequency components less than the predetermined frequency are removed from the frequency components contained in the relative change amount ($\Delta O_2$Hb). Normally, the cycle of concentration change due to chest compression (that is, the preferable compression cycle of the chest compression process) is shorter than the cycles of the primary concentration changes in the state where various procedures are being performed on a cardiopulmonary arrest person.

Therefore, by removing the low frequency components (that is, the long cycle components) from the measured relative change amount ($\Delta O_2$Hb) as in the concentration measurement apparatus 1 and the concentration measurement method according to the present embodiment, information on the concentration change due to chest compression can be extracted favorably. Based on this information, a chest compression performer can objectively judge whether or not chest compression is being performed appropriately. It thus becomes possible for the performer to perform or maintain chest compression more appropriately.

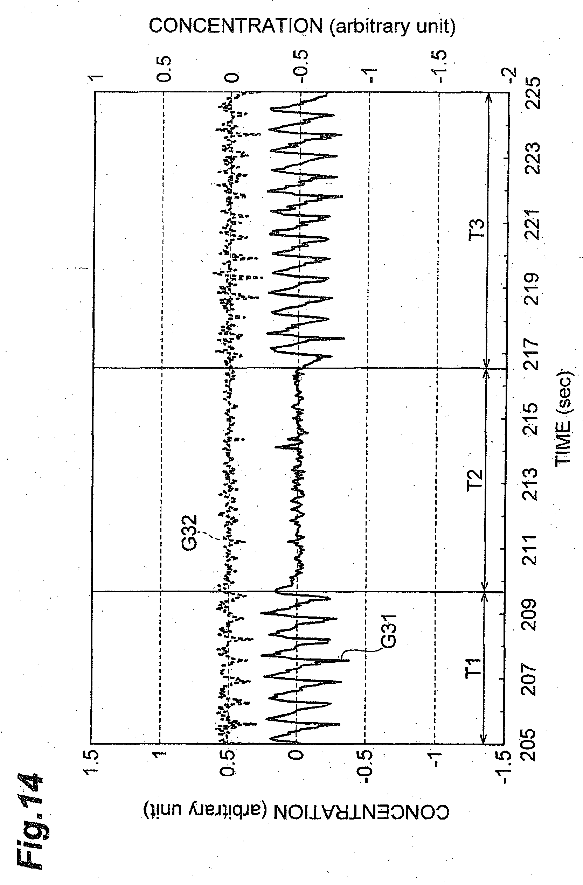

Also, as described above, although it is desirable for chest compression to be performed continuously, it may have to be interrupted due to unavoidable matters, such as a necessary procedure, change of chest compression performer, etc. In such cases, it is desired that the chest compression performer, etc., be able to check the brain oxygenation state during the interruption of chest compression. Here, FIG. 14 is a graph of compression time series data when chest compression is interrupted. In FIG. 14, periods T1 and T3 are periods in which chest compression is being performed, and a period T2 is a period in which chest compression is interrupted. Also, a graph G31 indicates time series data of the filtering-processed oxygenated hemoglobin concentration temporal relative change amount ($\Delta O_2$Hb), and a graph G32 indicates time series data of the filtering-processed deoxygenated hemoglobin concentration temporal relative change amount ($\Delta$HHb).

As shown in FIG. 14, due to the filtering process, the waveforms in the periods T1 and T3 accurately express the changes of the temporal relative change amounts ($\Delta O_2$Hb, $\Delta$HHb) due to chest compression. However, the long-cycle concentration change components that express the brain oxygenation state are removed, and therefore, the variations of the temporal relative change amounts ($\Delta O_2$Hb, $\Delta$HHb) are also extremely small in the period T2 in which chest compression is interrupted. It is thus difficult for the performer, etc., to check the brain oxygenation state during the interruption of chest compression.

Therefore, with the concentration measurement apparatus 1 and the concentration measurement method according to the present embodiment, first, the CPU 14 judges whether or not chest compression is being performed. Then, if chest compression is not performed for the predetermined time, the display section 15 switches from displaying the compression time series data that indicate the relative change amount from which the long cycle components have been removed (that is, indicate the concentration variation due to chest compression) to displaying the interruption time series data that indicate the relative change amount containing the long cycle components (that is, mainly indicate the brain oxygenation state) (see FIG. 11). The performer, etc., can thereby easily check the brain oxygenation state during the interruption of chest compression. Also, if a change is not seen in the oxygenated hemoglobin concentration temporal relative change amount ($\Delta O_2$Hb) during the interruption of chest compression, there is a possibility that the brain metabolism is significantly lowered. Precious information is thus provided in such a context as well.

Also, in switching from displaying the compression time series data to displaying the interruption time series data, the display section 15 may retrospectively display interruption time series data obtained before the CPU 14 recognized that chest compression is not performed for the predetermined time. With the concentration measurement apparatus 1 and the concentration measurement method according to the present embodiment, it is judged that chest compression is interrupted when the chest compression is not performed for the predetermined time. Therefore, at the point at which this judgment is made, some amount of time will already have elapsed from the interruption of chest compression, and it is preferable for the chest compression performer, etc., to be able to check the brain oxygenation state in this interval. With the concentration measurement apparatus 1 and the concentration measurement method described above, the display section 15 retrospectively displays interruption time series data obtained before it is recognized that chest compression is not performed for the predetermined time, so that the performer, etc., can easily check the brain oxygenation state in the interval from the interruption of chest compression to the recognition of the interruption. More preferably, the interruption time series data are displayed retrospectively back to the point at which it was first recognized that chest compression is not performed.

Also, as in the present embodiment, the CPU 14 preferably further determines the temporal relative change amount of at least one of the total hemoglobin concentration and the deoxygenated hemoglobin concentration ($\Delta$cHb, $\Delta$HHb), in addition to the oxygenated hemoglobin concentration temporal relative change amount ($\Delta O_2$Hb). Further, preferably, the same processes as those performed on the oxygenated hemoglobin concentration temporal relative change amount ($\Delta O_2$Hb) are performed on these relative change amounts ($\Delta$cHb, $\Delta$HHb), to display the compression time series data thereof on the display section 15, and switch to the display of the interruption time series data on the display section 15 when chest compression is interrupted. The judging of whether or not chest compression is being performed appropriately and the check of the brain oxygenation state during the interruption of chest compression can thereby be performed more accurately.

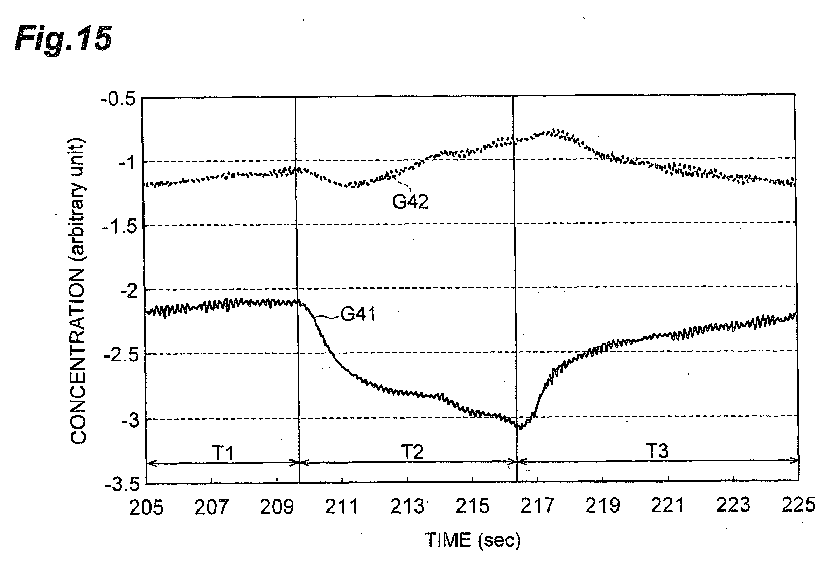

FIG 15 is a graph of interruption time series data displayed on the display section 15 as a modified example of the above-described embodiment. The interruption time series data shown in FIG. 15 are data indicating amounts obtained by subtracting the filtering-processed temporal relative change amounts from the non-filtering-processed temporal relative change amounts (that is, data from which the variation components due to chest compression have been removed). In FIG. 15, periods T1 and T3 are periods in which chest compression is being performed, and a period T2 is a period in which chest compression is interrupted. Also, a graph G41 indicates time series data of the filtering-processed oxygenated hemoglobin concentration temporal relative change amount ($\Delta O_2$Hb), and a graph G42 indicates time series data of the filtering-processed deoxygenated hemoglobin concentration temporal relative change amount ($\Delta$HHb).

The display section 15 may display such data, from which the variation components due to chest compression have been removed, as the interruption time series data when chest compression is interrupted. The brain oxygenation state during the interruption of chest compression can thereby be indicated to the performer, etc., more effectively.

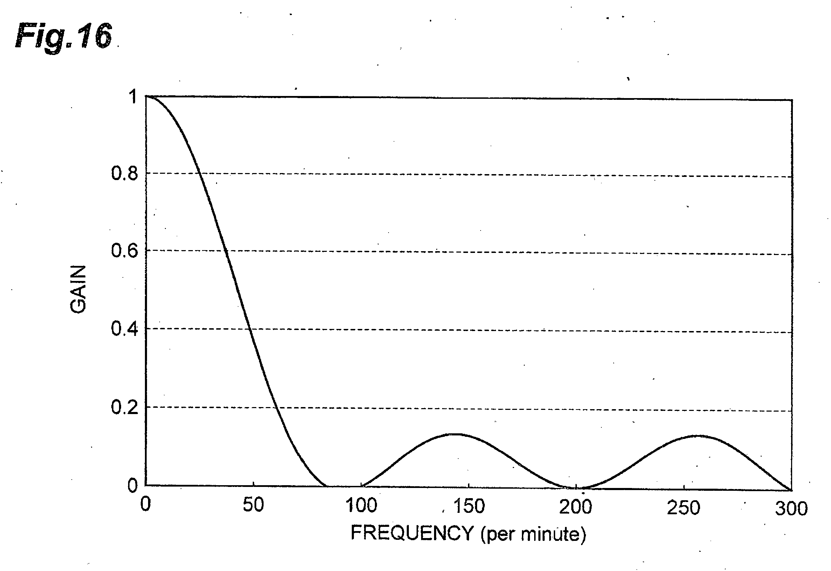

FIG. 16 is a diagram of frequency characteristics of a low-pass filter. The interruption time series data may be prepared using a low-pass filter such as that shown in the figure to take out variation components of a low frequency range. Here, the cutoff frequency of this low-pass filter is lower than the repetition frequency of a general chest compression (to give one example, 100 times/minute).

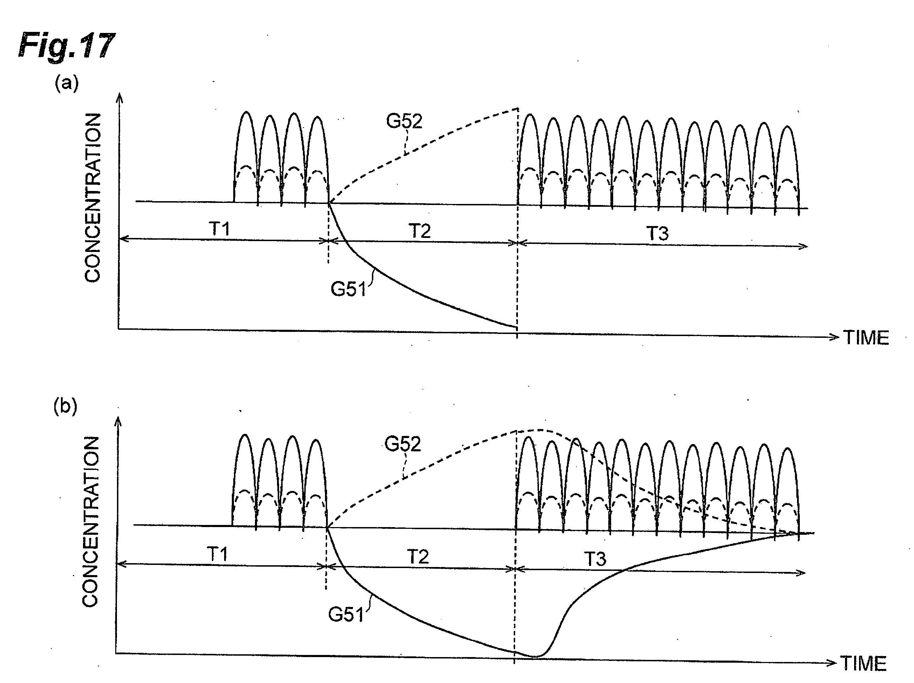

(a) in FIG. 17 and (b) in FIG. 17 are graphs that schematically show time series data displayed on the display section 15. In each of (a) in FIG. 17 and (b) in FIG. 17, a graph G51 indicates time series data of the oxygenated hemoglobin concentration temporal relative change amount ($\Delta O_2$Hb), and a graph G52 indicates time series data of the deoxygenated herrioglobin concentration temporal relative change amount ($\Delta$HHb).

(a) in FIG 17 shows the contents displayed on the display section 15 of the above-described embodiment. That is, the compression time series data are displayed in a period T1 in which chest compression is performed, and the compression time series data are not displayed but the interruption time series data are displayed in a period T2 in which chest compression is interrupted. Further, the display of the interruption time series data is stopped and the compression time series data are displayed again, when chest compression is restarted in a period T3.

On the other hand, (b) in FIG. 17 shows a modified example of the contents displayed on the display section 15 of the above-described embodiment. This modified example is the same as the example shown in (a) in FIG 17 in regard to the compression time series data being displayed in the period T1 in which chest compression is performed, and the compression time series data not being displayed but the interruption time series data being displayed in the period T2 in which chest compression is interrupted. However, when chest compression is restarted in the period T3, the display of the interruption time series data is not stopped, and the interruption time series data and the compression time series data are displayed overlappingly. By the display section 15 performing such a display, the manner of recovery of the brain oxygenation state after chest compression is restarted can be checked easily.

The concentration measurement apparatus and the concentration measurement method according to the present invention is not restricted to the embodiment described above, and various modifications are possible. For example, although with the concentration measurement apparatus 1 and the concentration measurement method according to the above-described embodiment, the respective relative change amounts ($\Delta$cHb, $\Delta O_2$Hb, $\Delta$HHb) of the total hemoglobin concentration, oxygenated hemoglobin concentration, and deoxygenated hemoglobin concentration are determined, with the concentration measurement apparatus and concentration measurement method according to the present invention, material for making an objective judgment related to whether or not chest compression is being performed appropriately and related to the transition of the brain oxygenation state when chest compression is interrupted can be indicated by determining at least the oxygenated hemoglobin concentration relative change amount ($\Delta O_2$Hb).

Also, the filtering process in the concentration measurement apparatus and concentration measurement method according to the present invention is not restricted to those given as examples in the embodiment, and any filtering process capable of removing frequency components less than a predetermined frequency $f_0$ from the relative change amounts ($\Delta cHb$, $\Delta O_2Hb$) may be used favorably in the present invention.

Also, with the present invention, the hemoglobin oxygen saturation (TOI), determined by near-infrared spectral analysis in a manner similar to the respective relative change amounts ($\Delta cHb$, $\Delta O_2Hb$, $\Delta HHb$) of the total hemoglobin concentration, oxygenated hemoglobin concentration, and deoxygenated hemoglobin concentration, may be displayed in a graph or as a numerical value together with the relative change amounts on the display section. Improvement of the brain oxygen state by the chest compression can thereby be checked to maintain the motivation of the chest compression performer. The TOI may be an average value for a predetermined duration (for example, 5 seconds).

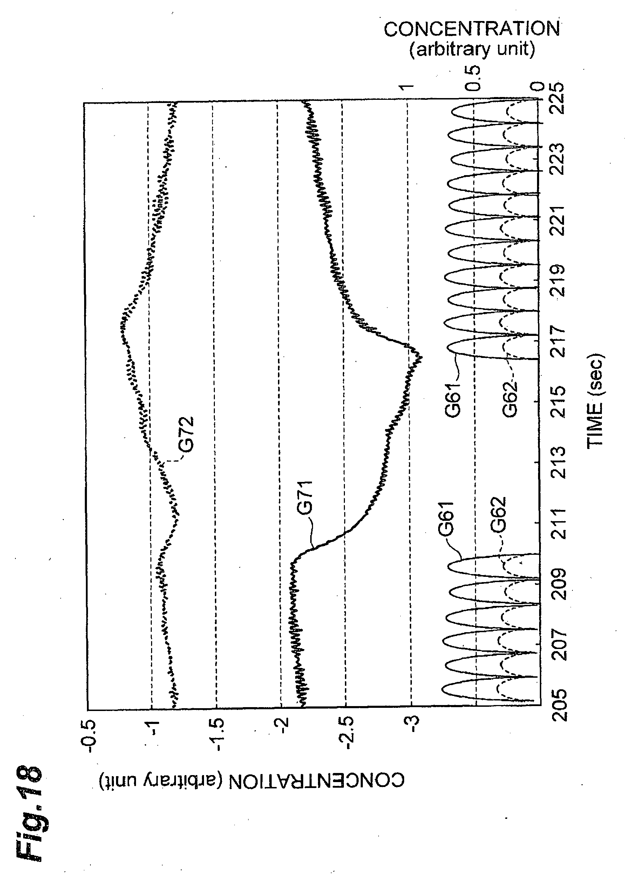

Also, with the concentration measurement apparatus and the concentration measurement method according to the present invention, the display section may, as shown in FIG. 18, display time series data, from which the frequency components less than the predetermined frequency $f_0$ have been removed by the filtering process (graphs G61 and G62), together with time series data containing the frequency components less than the predetermined frequency $f_0$ (graphs G71 and G72). In FIG 18, graphs G61 and G71 indicate the oxygenated hemoglobin concentration relative change amount ($\Delta O_2Hb$), and graphs G62 and G72 indicate the deoxygenated hemoglobin concentration relative change amount ($\Delta HHb$).

Also, with the concentration measurement apparatus and the concentration measurement method according to the present invention, a warning display may be displayed on the display section or a warning sound may be generated, when the calculation section judges that chest compression is interrupted.

Also, with the concentration measurement apparatus and the concentration measurement method according to the present invention, the display section may have a function by which display of time series data, from which the frequency components less than the predetermined frequency $f_0$ have been removed by the filtering process, and display of time series data containing the frequency components less than the predetermined frequency $f_0$ are switched manually, regardless of whether or not chest compression is being performed.

Also, with the concentration measurement apparatus and the concentration measurement method according to the present invention, the display section may further perform display of the cumulative number of times of chest compression performed from the start of measurement, display of the cumulative time of periods during which chest compression is not performed for no less than a predetermined time (for example, no less than 4 seconds), display of the time of start of measurement and the TOI value at that time, and display of the time elapsed from the start of measurement, etc.

The concentration measurement apparatus according to the embodiment is a concentration measurement apparatus for measuring a temporal relative change amount of oxygenated hemoglobin concentration, that varies due to repetition of chest compression, in a head, and has a configuration including a light incidence section irradiating the head with measurement light, a light detection section detecting the measurement light that has propagated through the interior of the head and generating a detection signal in accordance with the intensity of the detected measurement light, a calculation section determining, based on the detection signal, the temporal relative change amount of the oxygenated hemoglobin concentration and performing a filtering process of removing frequency components less than a predetermined frequency from frequency components contained in the relative change amount, and a display section displaying first time series data indicating the filtering-processed relative change amount of the oxygenated hemoglobin concentration, and where the calculation section judges, based on the detection signal, whether or not chest compression is being performed and, if chest compression is not performed for a predetermined time, the display section switches from displaying the first time series data to displaying second time series data indicating the temporal relative change amount of the oxygenated hemoglobin concentration that contains frequency components less than the predetermined frequency.

Also, the concentration measurement method according to the embodiment is a concentration measurement method of measuring a temporal relative change amount of oxygenated hemoglobin concentration, that varies due to repetition of chest compression, in a head, and has a configuration including a light incidence step of irradiating the head with measurement light, a light detection step of detecting the measurement light that has propagated through the interior of the head and generating a detection signal in accordance with the intensity of the detected measurement light, a calculation step of determining, based on the detection signal, the temporal relative change amount of the oxygenated hemoglobin concentration and performing a filtering process of removing frequency components less than a predetermined frequency from frequency components contained in the relative change amount, and a display step of displaying first time series data indicating the filtering-processed relative change amount of the oxygenated hemoglobin concentration, and where, in the calculation step, whether or not chest compression is being performed is judged based on the detection signal and, if chest compression is not performed for a predetermined time, switching from displaying the first time series data to displaying second time series data indicating the temporal relative change amount of the oxygenated hemoglobin concentration that contains frequency components less than the predetermined frequency is performed in the display step.

The "filtering process of removing frequency components less than a predetermined frequency" in the above-described concentration measurement apparatus and the concentration measurement method refers to a process of decreasing the proportion of frequency components less than the predetermined frequency until the frequency component due to chest compression appears at a sufficiently recognizable level, and is not restricted to completely removing the frequency components less than the predetermined frequency.

Also, the concentration measurement apparatus may have a configuration further including a storage section storing the second time series data or data corresponding to the second time series data, and where, in switching from displaying the first time series data to displaying the second time series data, the display section retrospectively displays the second time series data obtained before it is recognized that chest compression is not performed for the predetermined time. With the above-described concentration measurement apparatus, it is recognized that chest compression is interrupted when the chest compression is not performed for the predetermined time. Therefore, at the point at which this judgment is made, some amount of time will already have elapsed from the interruption of chest compression, and it is preferable for the chest compression performer, etc., to be able to check the brain oxygenation state in this interval as well. With this concentration measurement apparatus, the display section retrospectively displays second time series data obtained before it is recognized that chest compression is not performed for the predetermined time, so that the performer, etc., can easily check the brain oxygenation state in the interval from the interruption of chest compression to the recognition of the interruption.

Also, the concentration measurement apparatus may have a configuration where the calculation section further determines, based on the detection signal, the temporal relative change amount of at least one of the total hemoglobin concentration and the deoxygenated hemoglobin concentration and further performs a filtering process of removing frequency components less than a predetermined frequency from frequency components contained in the relative change amount, the display section further displays third time series data indicating the filtering-processed relative change amount of at least one of the total hemoglobin concentration and the deoxygenated hemoglobin concentration, and if chest compression is not performed for a predetermined time, the display section switches from displaying the third time series data to displaying fourth time series data indicating the temporal relative change amount of at least one of the total hemoglobin concentration and the deoxygenated hemoglobin concentration that contains frequency components less than the predetermined frequency. By the display section thus displaying not only the oxygenated hemoglobin concentration but also the total hemoglobin concentration or the deoxygenated hemoglobin concentration, the judging of whether or not chest compression is being performed appropriately and the check of the brain oxygenation state during the interruption of chest compression can be performed more accurately.

Also, the concentration measurement apparatus may have a configuration where the second time series data are data indicating an amount obtained by subtracting the filtering-processed oxygenated hemoglobin concentration temporal relative change amount from the non-filtering-processed oxygenated hemoglobin concentration temporal relative change amount. The brain oxygenation state during interruption of chest compression can thereby be indicated more effectively to a performer, etc.

Industrial Applicability

The present invention can be used as a concentration measurement apparatus and a concentration measurement method that enable a chest compression performer, etc., to easily check the brain oxygenation state during interruption of chest compression.

Reference Signs List

1 - concentration measurement apparatus, 10 - main unit section, 11 - light emitting section, 12 - sample hold circuit, 13 - converter circuit, 14 - calculation section, 15 - display section, 18 - data bus, 20 - probe, 21 - light incidence section, 22 - light detection section, 23 - holder, 24 - optical fiber, 25 - prism, 26 - photodetection element, 27 - preamplifier section, 28 - cable.

**Claims**

1. A concentration measurement apparatus for measuring a temporal relative change amount of oxygenated hemoglobin concentration, that varies due to repetition of chest compression, in a head, comprising:

   a light incidence section making measurement light incident on the head;
   a light detection section detecting the measurement light that has propagated through the interior of the head and generating a detection signal in accordance with the intensity of the detected measurement light;
   a calculation section determining, based on the detection signal, the temporal relative change amount of the oxygenated hemoglobin concentration and performing a filtering process of removing frequency components less than a predetermined frequency from frequency components contained in the relative change amount; and a display section displaying first time series data indicating the filtering-processed relative change amount of the oxygenated hemoglobin concentration, wherein

the calculation section judges, based on the detection signal, whether or not chest compression is being performed and, if chest compression is not performed for a predetermined time, the display section switches from displaying the first time series data to displaying second time series data indicating the temporal relative change amount of the oxygenated hemoglobin concentration that contains frequency components less than the predetermined frequency.

2. The concentration measurement apparatus according to Claim 1, further comprising a storage section storing the second time series data or data corresponding to the second time series data, wherein
in switching from displaying the first time series data to displaying the second time series data, the display section retrospectively displays the second time series data obtained before it is recognized that chest compression is not performed for the predetermined time.

3. The concentration measurement apparatus according to Claim 1 or 2, wherein the calculation section further determines, based on the detection signal, the temporal relative change amount of at least one of the total hemoglobin concentration and the deoxygenated hemoglobin concentration and further performs a filtering process of removing frequency components less than the predetermined frequency from frequency components contained in the relative change amount,
the display section further displays third time series data indicating the filtering-processed relative change amount of at least one of the total hemoglobin concentration and the deoxygenated hemoglobin concentration, and
if chest compression is not performed for a predetermined time, the display section switches from displaying the third time series data to displaying fourth time series data indicating the temporal relative change amount of at least one of the total hemoglobin concentration and the deoxygenated hemoglobin concentration that contains frequency components less than the predetermined frequency.

4. The concentration measurement apparatus according to any one of Claims 1 to 3, wherein the second time series data are data indicating an amount obtained by subtracting the filtering-processed oxygenated hemoglobin concentration temporal relative change amount from the non-filtering-processed oxygenated hemoglobin concentration temporal relative change amount.

5. A concentration measurement method of measuring a temporal relative change amount of oxygenated hemoglobin concentration, that varies due to repetition of chest compression, in a head, the method comprising:

a light incidence step of making measurement light incident on the head;
a light detection step of detecting the measurement light that has propagated through the interior of the head and generating a detection signal in accordance with the intensity of the detected measurement light;
a calculation step of determining, based on the detection signal, the temporal relative change amount of the oxygenated hemoglobin concentration and performing a filtering process of removing frequency components less than a predetermined frequency from frequency components contained in the relative change amount; and
a display step of displaying first time series data indicating the filtering-processed relative change amount of the oxygenated hemoglobin concentration, wherein

in the calculation step, whether or not chest compression is being performed is judged based on the detection signal and, if chest compression is not performed for a predetermined time, switching from displaying the first time series data to displaying second time series data indicating the temporal relative change amount of the oxygenated hemoglobin concentration that contains frequency components, less than the predetermined frequency is performed in the display step.

**Patentansprüche**

1. Konzentrationsmessvorrichtung zum Messen einer zeitweiligen relativen Änderungsmenge der oxygenierten Hämoglobinkonzentration, die auf Grund der Brustkorbkompression variiert, in einem Kopf, umfassend:

einen Lichteinfallabschnitt, der das Messlicht auf den Kopf einfallen lässt;
einen Lichtnachweisabschnitt, der das Messlicht nachweist, das sich durch das Innere des Kopfes ausgebreitet hat, und der ein Nachweissignal entsprechend der Intensität des festgestellten Messlichtes erzeugt;
einen Berechnungsabschnitt, der auf der Basis des Nachweissignals die zeitweilige relative Änderungsmenge der oxygenierten Hämoglobinkonzentration bestimmt, und einen Filterprozess zum Entfernen von Frequenzkomponenten, die kleiner als eine vorgegebene Frequenz sind, aus den Frequenzkomponenten ausführt, die in der relativen Änderungsmenge enthalten sind; und
einen Anzeigeabschnitt, der erste Seriendaten anzeigt, die die durch den Filterprozess verarbeitete relative Änderungsmenge der oxygenierten Hämoglobinkonzentration angibt, wobei
der Berechnungsabschnitt auf der Basis des Nachweissignals beurteilt, ob eine Brustkompression ausgeführt wird oder nicht, und, wenn eine Brustkompression für eine vorgegebene Zeit nicht ausgeführt wird, schaltet der Anzeigeabschnitt von der Anzeige der ersten Seriendaten zur Anzeige von zweiten Seriendaten, die die zeitweilige relative Änderungsmenge der oxygenierten Hämoglobinkonzentration angeben, welche Frequenzkomponenten enthält, die kleiner als die vorgegebene Frequenz sind.

2. Konzentration Messvorrichtung nach Anspruch 1, die ferner einen Speicherabschnitt umfasst, der die zweiten Seriendaten speichert oder Daten, die den zweiten Seriendaten entsprechen, wobei
beim Schalten von der Anzeige der ersten Seriendaten zur Anzeige der zweiten Seriendaten der Anzeigeabschnitt retrospektiv die zweiten Seriendaten anzeigt, die gewonnen wurden, bevor erkannt wurde, dass keine Brustkompression für die vorgegebene Zeit ausgeführt wird.

3. Konzentrationsmessvorrichtung nach Anspruch 1 oder 2, wobei der Berechnungsabschnitt ferner auf der Basis des Nachweissignals die temporale relative Änderungsmenge von mindestens einem Element aus der gesamten Hämoglobinkonzentration und der deoxygenierten Hämoglobinkonzentration bestimmt und ferner einen Filterprozess zum Entfernen von Frequenzkomponenten, die kleiner als die vorgegebene Frequenz sind, aus Frequenzkomponenten ausführt, die in der relativen Änderungsmenge enthalten sind,
der Anzeigeabschnitt ferner dritte Seriendaten anzeigt, die die filterverarbeitete relative Änderungsmenge von mindestens einem Element aus der totalen Hämoglobinkonzentration und der deoxygenierten Hämoglobinkonzentration angeben, und
wenn keine Brustkompression über eine vorgegebene Zeit ausgeführt wird, schaltet der Anzeigeabschnitt von der Anzeige der dritten Seriendaten auf die Anzeige von vierten Seriendaten um, die die temporale relative Änderungsmenge von mindestens einem Element aus der totalen Hämoglobinkonzentration und der deoxygenierten Hämoglobinkonzentration angeben, die Frequenzkomponenten enthalten, welche kleiner als die vorgegebene Frequenz sind.

4. Konzentrationsmessvorrichtung nach einem der Ansprüche 1 bis 3, wobei die zweiten Seriendaten Daten sind, die eine Menge angeben, welche durch Subtrahieren der temporalen relativen Änderungsmenge der filterverarbeiteten oxygenierten Hämoglobinkonzentration von der temporalen relativen Änderungsmenge der nichtfilterverarbeiteten oxygenierten Hämoglobinkonzentration gewonnen wird.

5. Konzentrationsmessverfahren zum Messen einer temporalen relativen Änderungsmenge der oxygenierten Hämoglobinkonzentration, aufgrund von Brustkompression, Wiederholung von Brustkompression, in einem Kopf variiert, wobei das Verfahren Folgendes umfasst:

einen Lichteinfallschritt, der das Messlicht auf den Kopf einfallen lässt;
einen Lichtnachweisschritt zum Nachweisen des Messlichts, das sich durch das Innere des Kopfes ausgebreitet hat, und zum Erzeugen eines Nachweissignals entsprechend der Intensität des festgestellten Messlichtes;
einen Berechnungsschritt zum Bestimmen auf der Basis des Nachweissignals der temporalen relativen Änderungsmenge der oxygenierten Hämoglobinkonzentration und zum Ausführen eines Filterprozesses zum Entfernen von Frequenzkomponenten, die kleiner als eine vorgegebene Frequenz sind, aus den Frequenzkomponenten, die in der relativen Änderungsmenge enthalten sind; und
einen Anzeigeschritt zum Anzeigen der ersten Seriendaten, der die filterverarbeitete relative Änderungsmenge der oxygenierten Hämoglobinkonzentration angibt, wobei
im Berechnungsschritt auf der Basis des Nachweissignals beurteilt wird, ob eine Brustkompression ausgeführt wird oder nicht, und, wenn keine Brustkompression über eine vorgegebene Zeit ausgeführt wird, wird das Umschalten der Anzeige der ersten Seriendaten zur Anzeige von zweiten Seriendaten, die die temporale relative Änderungsmenge der oxygenierten Hämoglobinkonzentration angeben, welche Frequenzkomponenten enthält, die kleiner als die vorgegebene Frequenz sind, im Anzeigeschritt vorgenommen.

**Revendications**

1. Appareil de mesure de concentration pour mesurer une ampleur de changement relatif temporel de la concentration en hémoglobine oxygénée, qui varie en fonction de la répétition de la compression thoracique, dans une tête, comprenant :

    une section d'incidence de lumière rendant la lumière de mesure incidente sur la tête ;
    une section de détection de lumière détectant la lumière de mesure qui a été propagée dans l'intérieur de la tête et produisant un signal de détection en fonction de l'intensité de la lumière de mesure détectée ;
    une section de calcul déterminant, sur la base du signal de détection, l'ampleur de changement relatif temporel de la concentration en hémoglobine oxygénée et effectuant un processus de filtrage de retrait de composantes de fréquence inférieures à une fréquence prédéterminée des composantes de fréquence contenues dans l'ampleur de changement relatif ; et
    une section d'affichage affichant des premières données de séries temporelles indiquant l'ampleur de changement relatif traitée par filtrage de la concentration en hémoglobine oxygénée, où
    la section de calcul estime, sur la base du signal de détection, si une compression thoracique est ou non effectuée et, si une compression thoracique n'est pas effectuée pendant une durée prédéterminée, la section d'affichage passe d'un affichage des premières données de séries temporelles à un affichage de deuxièmes données de séries temporelles indiquant l'ampleur de changement relatif temporel de la concentration en hémoglobine oxygénée qui contient des composantes de fréquence inférieures à la fréquence prédéterminée.

2. Appareil de mesure de concentration selon la revendication 1, comprenant en outre une section de stockage stockant les deuxièmes données de séries temporelles ou des données correspondant aux deuxièmes données de séries temporelles, où
    en passant de l'affichage des premières données de séries temporelles à l'affichage des deuxièmes données de séries temporelles, la section d'affichage affiche rétrospectivement les deuxièmes données de séries temporelles obtenues avant qu'il soit reconnu que la compression thoracique n'est pas effectuée pendant la durée prédéterminée.

3. Appareil de mesure de concentration selon la revendication 1 ou 2, où la section de calcul détermine en outre, sur la base du signal de détection, l'ampleur de changement relatif temporel d'au moins l'une de la concentration en hémoglobine totale et de la concentration en hémoglobine désoxygénée et effectue en outre un processus de filtrage de retrait des composantes de fréquence inférieures à la fréquence prédéterminée des composantes de fréquence contenues dans l'ampleur de changement relatif,
    la section d'affichage affiche en outre des troisièmes données de séries temporelles indiquant l'ampleur de changement relatif traitée par filtrage d'au moins l'une de la concentration en hémoglobine totale et de la concentration en hémoglobine désoxygénée, et
    si une concentration thoracique n'est pas effectuée pendant une durée prédéterminée, la section d'affichage passe de l'affichage des troisièmes données de séries temporelles à l'affichage de quatrièmes données de séries temporelles indiquant l'ampleur de changement relatif temporel d'au moins l'une de la concentration en hémoglobine totale et de la concentration en hémoglobine désoxygénée qui contient des composantes de fréquence inférieures à la fréquence prédéterminée.

4. Appareil de mesure de concentration selon l'une quelconque des revendications 1 à 3, où les deuxièmes données de séries temporelles sont des données indiquant une ampleur obtenue en soustrayant l'ampleur de changement relatif temporel de la concentration en hémoglobine oxygénée traitée par filtrage de l'ampleur de changement relatif temporel de la concentration en hémoglobine oxygénée non traitée par filtrage.

5. Procédé de mesure de concentration pour mesurer une ampleur de changement relatif temporel d'une concentration en hémoglobine oxygénée, qui varie en fonction de la répétition d'une compression thoracique, dans une tête, le procédé comprenant :

    une étape d'incidence de lumière rendant la lumière de mesure incidente sur la tête ;
    une étape de détection de la lumière détectant la lumière de mesure qui a été propagée dans l'intérieur de la tête et produisant un signal de détection en fonction de l'intensité de la lumière de mesure détectée ;
    une étape de calcul de détermination, sur la base du signal de détection, de l'ampleur de changement relatif temporel de la concentration en hémoglobine oxygénée et d'exécution d'un processus de filtrage de retrait des composantes de fréquence inférieures à une fréquence prédéterminée des composantes de fréquence contenues dans l'ampleur de changement relatif ; et

une étape d'affichage affichant des premières données de séries temporelles indiquant l'ampleur de changement relatif traitée par filtrage de la concentration en hémoglobine oxygénée, où

dans l'étape de calcul, le fait qu'une compression thoracique soit ou non effectuée est évalué sur la base du signal de détection et, si une compression thoracique n'est pas effectuée pendant une durée prédéterminée, le passage de l'affichage des premières données de séries temporelles à l'affichage de deuxièmes données de séries temporelles indiquant l'ampleur de changement relatif temporel de la concentration en hémoglobine oxygénée qui contient des composantes de fréquence inférieures à la fréquence prédéterminée est effectué dans l'étape d'affichage.

*Fig.1*

ΔHHb

ΔO₂Hb

EP 2 818 109 B1

*Fig.2*

(a)

20

21
24    25

26

II

23

22

II

(b)

20

28

22  27

LIGHT →

24    25

23

26

21

**Fig.3**

EP 2 818 109 B1

# Fig.4

```
                    ┌─────────────┐
                    │    START    │
                    └──────┬──────┘
                           │
        ┌──────────────────┴──────────────────┐
        │  INCIDENCE OF MEASUREMENT LIGHT      │──S11
        │            INTO HEAD                 │
        └──────────────────┬──────────────────┘
                           │
        ┌──────────────────┴──────────────────┐
        │  DETECTION OF MEASUREMENT LIGHT      │──S12
        │          EMITTED FROM HEAD           │
        └──────────────────┬──────────────────┘
                           │
        ┌──────────────────┴──────────────────┐
        │ CALCULATION OF TEMPORAL RELATIVE     │──S13
        │ CHANGE AMOUNT OF HEMOGLOBIN          │
        │ CONCENTRATION                        │
        └──────────────────┬──────────────────┘
                           │
        ┌──────────────────┴──────────────────┐
        │       FILTERING PROCESS ON           │──S14
        │         CALCULATION RESULT           │
        └──────────────────┬──────────────────┘
                           │
        ┌──────────────────┴──────────────────┐
        │         DISPLAY OF SCREEN            │──S15
        └──────────────────┬──────────────────┘
                           │
                    ┌──────┴──────┐
                    │     END     │
                    └─────────────┘
```

EP 2 818 109 B1

# Fig.5

```
                    ┌─────────┐
                    │  START  │
                    └────┬────┘
                         │
          ┌──────────────▼──────────────┐
          │ JUDGE WHETHER OR NOT CHEST  │── S21
          │ COMPRESSION IS BEING        │
          │ PERFORMED                   │
          └──────────────┬──────────────┘
                         │
                    ╱────────╲  S22
                   ╱    IS     ╲      NO
                  ╱ CHEST       ╲──────────────┐
                  ╲ COMPRESSION ╱              │
                   ╲INTERRUPTED?╱              │
                    ╲──────────╱       ┌───────▼──────────────┐
                         │ YES         │ CONTINUE DISPLAY OF  │── S23
                         │             │ FILTERING-PROCESSED  │
                         │             │ DATA                 │
          ┌──────────────▼──────────────┐     └──────────────────────┘
          │ SWITCH TO DISPLAY OF        │
          │ DATA CONTAINING             │── S24
          │ COMPONENTS LESS THAN        │
          │ PREDETERMINED FREQUENCY     │
          └──────────────┬──────────────┘
                         │
          ┌──────────────▼──────────────┐
          │ JUDGE WHETHER OR NOT CHEST  │── S25
          │ COMPRESSION IS BEING        │
          │ PERFORMED                   │
          └──────────────┬──────────────┘
                         │
                    ╱────────╲  S26
           NO      ╱    IS     ╲
          ┌───────╱ CHEST       ╲
          │       ╲ COMPRESSION ╱
          │        ╲ RESTARTED? ╱
          │         ╲──────────╱
          │              │ YES
          │   ┌──────────▼──────────┐
          │   │ RESTART DISPLAY OF  │── S27
          │   │ FILTERING-PROCESSED │
          │   │ DATA                │
          │   └──────────┬──────────┘
          │              │
          │         ┌────▼────┐
          │         │   END   │
          │         └─────────┘
```

# Fig.6

(a)

(b)

EP 2 818 109 B1

Fig.7

Fig.8

EP 2 818 109 B1

Fig.9

**Fig.10**

(a)

(b)

Fig.11

# Fig.12

EP 2 818 109 B1

Fig.13

## Fig.14

*Fig.15*

EP 2 818 109 B1

## Fig.16

EP 2 818 109 B1

EP 2 818 109 B1

## Fig.17

(a)

(b)

Fig.18

EP 2 818 109 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **N. NAGDYMAN.** Cerebral oxygenation measured by near-infrared spectroscopy during circulatory arrest and cardiopulmonary resuscitation. *BRITISH JOURNAL OF ANAESTHESIA,* 01 September 2003, vol. 91 (3), 438-442

- **SUSUMU SUZUKI et al.** Tissue oxygenation monitor using NIR spatially resolved spectroscopy. *Proceedings of SPIE,* vol. 3597, 582-592